Europäisches Patentamt

European Patent Office

Office européen des brevets

(19)

(11) **EP 0 536 520 B1**

(12) **EUROPEAN PATENT SPECIFICATION**

(45) Date of publication and mention
of the grant of the patent:
**03.03.1999 Bulletin 1999/09**

(51) Int. Cl.$^6$: **A61K 38/21**

(21) Application number: **92114478.8**

(22) Date of filing: **10.10.1986**

(54) **Use of Human Interferon-Beta 2A for the preparation of medicaments**

Verwendung von menschlichem Interferon-Beta 2A für die Herstellung von Medikamenten

Utilisation de l'interféron-Beta 2A humain pour la préparation des medicaments

(84) Designated Contracting States:
**BE CH DE ES FR GB GR IT LI NL SE**

(30) Priority: **14.10.1985 IL 7671485**
**08.05.1986 US 860883**

(43) Date of publication of application:
**14.04.1993 Bulletin 1993/15**

(62) Document number(s) of the earlier application(s) in
accordance with Art. 76 EPC:
**86114049.9 / 0 220 574**

(60) Divisional application:
**97119173.9 / 0 835 661**
**94113232.6 / 0 645 452**

(73) Proprietor:
**YEDA RESEARCH AND DEVELOPMENT CO.
LTD.
Rehovot 76100 (IL)**

(72) Inventors:
• **Revel, Michael
Rehovot (IL)**

• **Zilberstein, Asher
Collegeville, PA 19426 (US)**

(74) Representative:
**Grünecker, Kinkeldey,
Stockmair & Schwanhäusser
Anwaltssozietät
Maximilianstrasse 58
80538 München (DE)**

(56) References cited:
**WO-A-88/00206**

• **PATENT ABSTRACTS OF JAPAN vol. 10, no. 13
(C-323)(2070) 18 January 1986**
• **PROCEEDINGS OF THE NATIONAL ACADEMY
OF SCIENCES OF USA. vol. 82, August 1985,
WASHINGTON US pages 5490 - 5494 TOSHIO
HIRANO ET AL 'Purification to homogeneity and
characterization of human B-cell differentiation
factor (BCDF or BSFp-2)'**

Remarks:
Divisional application 94113232.6 filed on 10/10/86.

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

**Description**

Interferon is an important antiviral and antitumor protein produced by the human body. Because of its species specificity, clinical use of interferon requires human interferon. Based on their neutralization by antibodies three types of interferon were defined which include interferon-alpha, produced mainly by leucocytes, interferon-beta, produced mainly by fibroblasts, and interferon-gamma, produced mainly by T-lymphocytes.

The major species of interferon produced by human fibroblasts in response to ds RNA is the 20 Kd glycoprotein IFN-$\beta_1$, encoded by a 0.9 kb RNA which is transcribed from an intron-less gene on chromosome 9. However, additional mRNAs which yield IFN activity when microinjected into frog oocytes have been observed in human fibroblasts induced by poly (rI)(rC) and by a sequential cycloheximide (CHX)-actinomycin D treatment. While cloning the IFN-$\beta_1$ cDNA, cDNA clones of another co-induced 1.3 kb RNA have been isolated which in reticulocyte lysates code for a 23-26 Kd polypeptide and in oocytes produce human IFN antiviral activity (J. Weissenbach et al. 1980 Proc. Natl. Acad. Sci. USA 77:7152-7156; British patent 2 063 882.)

Since this activity was inhibited by anti-IFN-$\beta_1$ antibodies, it was designated IFN-$\beta_2$. The protein product of IFN-$\beta_2$ RNA is immunologically distinct from IFN-$\beta_1$, although the biological activities of the two proteins are cross-neutralized by the same antibodies. The IFN-$\beta_2$ 1.3 kb RNA clearly originates from another gene than IFN-$\beta_1$, since IFN-$\beta_2$ RNA is formed in mouse-human hybrids lacking chromosome 9 (U. Nir (1984) Ph.D. Thesis, Weizmann Institute of Science, Rehovot, Israel).

In the German patent application P 30 43 981 a process is disclosed for the preparation of interferon-$\beta_2$ in purified form. Said interferon-$\beta_2$ is produced by isolating DNA containing the nucleotide sequence coding for interferon-$\beta_2$ in human cells after cultivating those cells which are able to produce interferon-$\beta_2$ when exposed to an inducer of interferon. mRNA from said induced cells is extracted and purified. Afterwards, the mRNA is transcribed into DNA and the DNA is cloned in a suitable vector.

It could be demonstrated that there is a second gene encoding IFN-$\beta_2$, which, when isolated and transfected into hamster or mouse cells produces human-specific IFN activity after induction by ds RNA and cycloheximide. The IFN produced has the properties ascribed to IFN-$\beta_2$ but does not have identical amino acid sequences, the proteins produced by the two genes are denominated IFN-$\beta_{2A}$ and IFN-$\beta_{2B}$ respectively.

IFN-$\beta_{2A}$ and its use in pharmaceutical compositions is also disclosed in WO 88/00206 (not pre-published). Further, JP-A-60169424 discloses the administration of BCDF, which is another designation for IFN-$\beta_{2A}$, to patients suffering from immune incompetence.

Therefore, it is an object of the present invention to provide further uses of IFN-$\beta_{2A}$ in pharmaceuticals.

According to the present invention, human interferon-$\beta_{2A}$ is used for preparing a medicament useful in the field of inflammation and/or acute phase response. According to preferred embodiments, the IFN-$\beta_{2A}$ is used for the preparation of a medicament inhibiting fibroblast proliferation or preventing sclerosis after infection.

The inventors were able to confirm the existence of biologically active human interferon-beta$_2$ (IFN-$\beta_2$) molecules and these molecules are produced by recombinant DNA technology. IFN-$\beta_2$ is a glycoprotein secreted by human fibroblasts which are induced by double-stranded (ds) RNA or viruses to produce interferon. IFN-$\beta_2$ amounts to about 5% of the total IFN activity produced by fibroblasts. The amino acid sequence of IFN-$\beta_2$, which was determined by cDNA sequencing, indicates only about 20% overall homology with that of the other human IFNs produced by such human cells (IFN-$\beta_1$ and IFN-$\alpha$s).

A human cDNA or gene encoding IFN-$\beta_{2A}$ may be cloned in accordance with the following description and fused to a strong viral transcriptional promoter. Hamster cells transformed by the fused cDNA or gene produce constitutively human-specific interferon activity which inhibits viral replication and cytopathic effect and induces the proteins typical of the biological response of human cells to interferons. A second gene encoding IFN-$\beta_2$ is also disclosed which, when isolated and transfected into hamster or mouse cells, produces human-specific IFN activity after induction by ds RNA and cycloheximide. As the IFN produced by both genes have the properties ascribed to IFN-$\beta_2$, but do not have identical amino acid sequences, the proteins produced by the two genes are denominated IFN-$\beta_{2A}$ and IFN-$\beta_{2B}$ respectively.

Description of the drawings

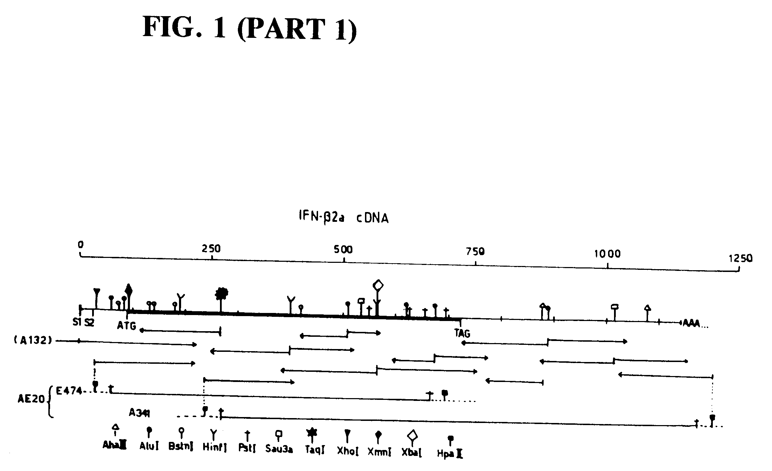

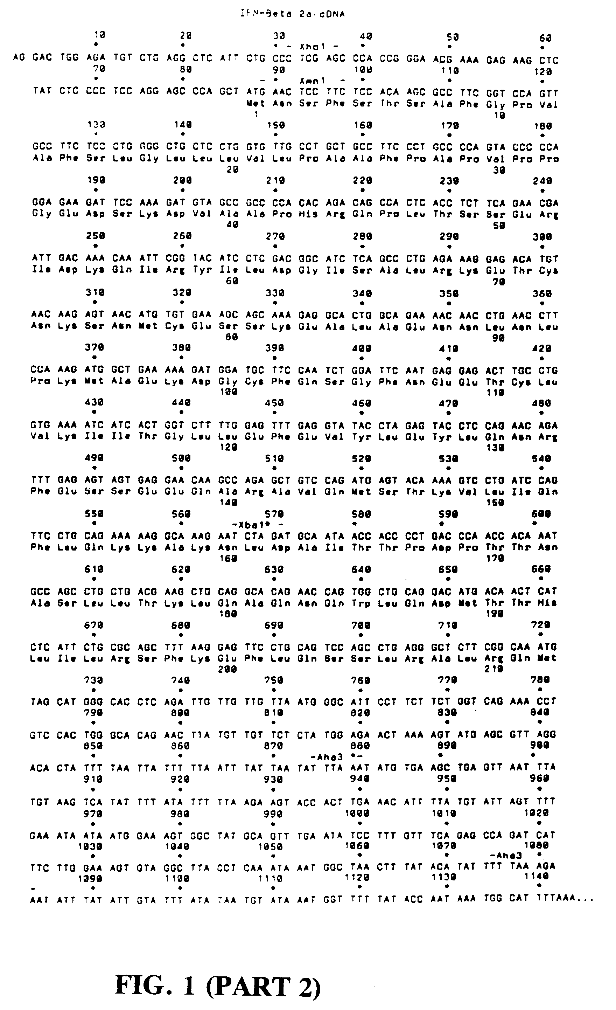

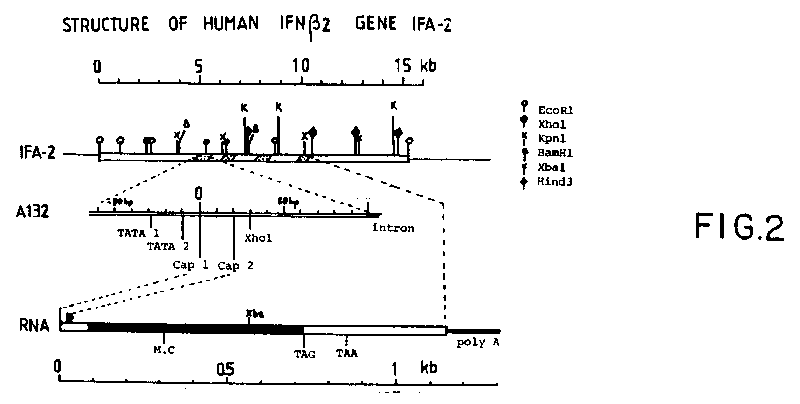

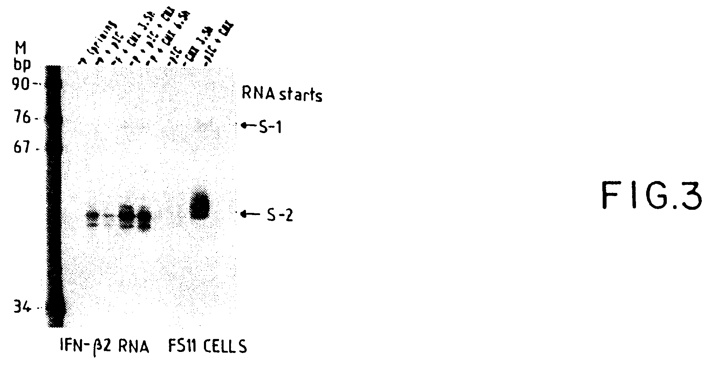

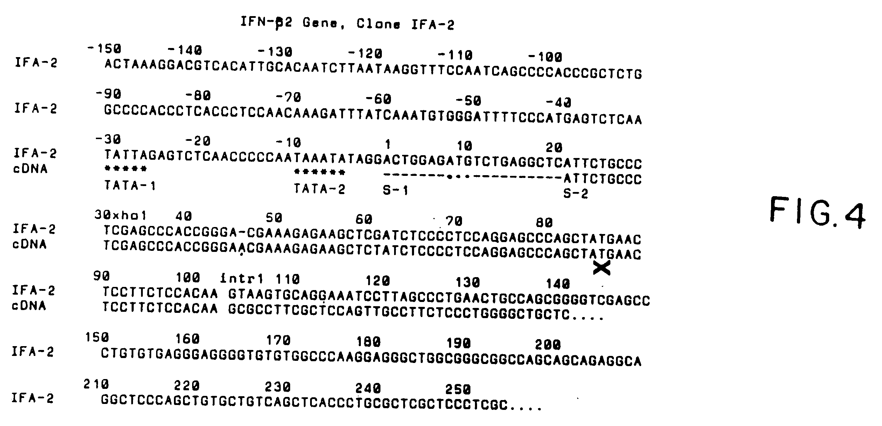

Figure 1 shows the restriction map, sequencing strategy and nucleotide sequence of IFN-$\beta_{2A}$ cDNA determined from clone AE20, formed by fusing E474 cDNA and A341 cDNA clones (Weissenbach et al. (1980), supra) by their Xba-1 site. The 5' end sequence was completed from the genomic clone IFA-2 shown in Fig. 2. Numbering begins from the S-1 start of Fig. 3 and 4. The amino acid sequence of the IFN-$\beta_{2A}$ protein is deduced.

Figure 2 shows the structure of genomic clone IFA-2 containing the IFN-$\beta_{2A}$ gene. A132 is a subclone of an EcoRI segment of IFA-2 in pBR322. Shaded areas are gene regions hybridizing to cDNA clones A341 and E474 (Weissenbach et al. (1980), supra). The positions of the two RNA starts (cap 1 and 2) and the TATA boxes are shown. In the RNA the longest open reading frame (ORF) of 212 amino acids (Fig. 1) is shown in black. M.C. indicates a second internal ATG in the same frame. No other long ORF are found in the entire IFN-$\beta_{2A}$ cDNA (Fig. 1).

Figure 3 shows the S1 nuclease analysis of 5' starts in IFN-$\beta_2$ RNA. The DNA probe, a fragment from subclone A132 of IFA-2 gene (Figure 2) labelled at BstN1 site was hybridized to total RNA from diploid fibroblasts treated (from left to right) by: priming with IFN-$\beta_1$ 200 U/ml, 16 hrs; priming and poly (rI)(rC)(pIC) 50 μg/ml, 3.5 hours; priming and cycloheximide (CHX) 50 μg/ml, 3.5 hours; priming and pIC plus CHX 3.5 hours; priming and CHX for 6.5 hours; no priming and pIC 3.5 hours; same CHX 3.5 hours; same pIC plus CHX 3.5 hours. S-1 and S-2 are the signals generated by the 2 RNA starts of Figure 2.

Figure 4 shows the sequence of the promoter region of the IFA-2 gene encoding IFN-$\beta_{2A}$. The sequence of part of subclone A132 (Figure 2) is shown compared to the IFN-$\beta_2$ cDNA. S1 and S2 are the 2 RNA starts determined in Figure 3. The initiator ATG is marked by X. The first intron starts at intr 1.

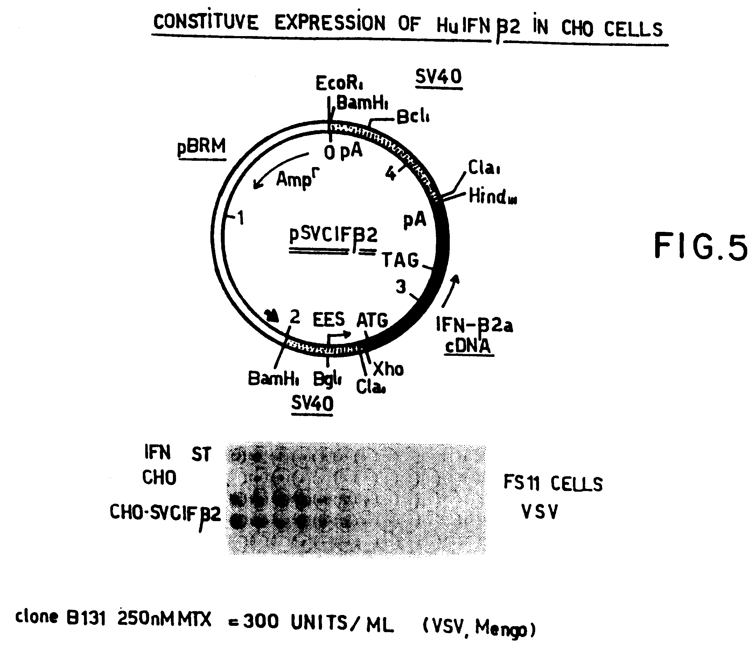

Figure 5 illustrates the fusion of IFN-$\beta_{2A}$ cDNA to the SV40 early promoter. A plasmid pSVIFA2-II was first constructed by subcloning the 5' Xho$_1$-BamH$_1$ (Xh-B) segment of the IFA-2 gene (from 5.5 kb to 7.5 kb in Figure 2) fused with a Cla$_1$-Xho$_1$ synthetic adaptor of 26 bp (restoring the 5' cDNA sequence), into a Cla$_1$ and Bam H$_1$-cut pBR plasmid. The 3' BamH$_1$-Hind$_{III}$ (B-H) gene segment (from 7.5 kb to 10.5 kb in Figure 2), subcloned in a a Hind$_{III}$, BamH$_I$-cut pBR plasmid, was excised using the Cla$_1$ site adjacent to Hind$_{III}$ in pBR322 and ligated to the above 5' segment to obtain the complete IFA-2 gene. A pSVE3 vector cut by Hind$_{III}$ as described in Y. Chernajovsky et al. (1984) DNA 3: 294-308, was reclosed with Cla$_1$ linkers(forming pSVC1a) and the above IFA-2 gene introduced in the Cla$_1$ site of pSVC1a in the same orientation as the SV40 early promoter forming pSVIFA2-II. A plasmid pSVIFA2-I was similarly constructed but the Xho$_1$ site of IFA-2 was fused directly to the Cla$_1$ site of pBR before introduction in pSVE3. To obtain the pSVCIFβ2 plasmid containing the IFN-$\beta_{2A}$ cDNA fused to the SV40 promoter, the above pSVIFA2-II DNA, cut by Xb$_1$, was partially cut by Xmn$_1$ to open the Xmn$_1$ site located 60 bp downstream from the Xho$_1$ site in the IFN-$\beta_2$ cDNA sequence (Figure 1), and not an Xmn$_1$ site located in the amp gene of the vector. To this construct, the Xmn$_1$-Xba$_1$ segment of IFN-$\beta_2$ cDNA clone AE20 (coordinates 92-566 in Fig. 1) was ligated, restoring the complete IFN-$\beta_{2A}$ cDNA sequence as shown in the Figure. EES is the early-early RNA start of SV40 T-ag gene; ATG is the IFN-$\beta_2$ initiation codon; pA are IFN-$\beta_2$ and SV40 polyadenylation sites, respectively. pSVCIFβ2 DNA was cotransfected with a pDHFR plasmid into CHO-K$_1$ DHFR$^-$ cells as in Y. Chernajovsky et al. (1984), (supra) and clones were tested for human IFN antiviral activity in FS11 cells using Vesicular Stomatitis Virus (VSV). Gene amplification was obtained by selecting CHO clones for methotrexate (MTX)-resistance. Assay of the culture medium of CHO-SVCIFβ2 B132-5M cells, resistant to 250 nM MTX, is shown in the lower part of the Figure. After VSV cytopathic effect developed, cells in the microplate were stained by methylene blue. Serial two-fold dilutions of the medium are from left to right. By comparison to IFN standard (ST) and non-transfected CHO cells, a titer of 300 U/ml rIFN-$\beta_2$ is calculated.

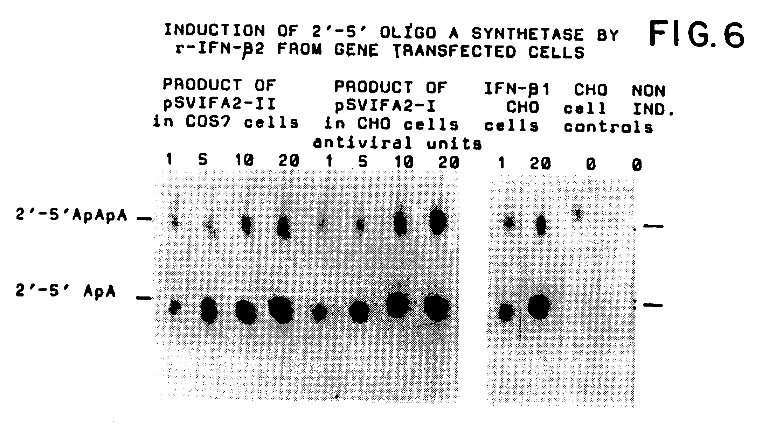

Figure 6 shows the results of an assay of (2'-5') oligo A synthetase with poly (rI)(rC)-agarose bound NP40 extracts (M. Revel et al. (1981) Meth. Enzymol. 79:149-161) of human FS11 fibroblasts previously exposed for 16 hours to medium from a) Cos7 cells two days post-transfection with the pSVIFA2-II DNA (described in Figure 5)(leftmost 4 lanes), b) hamster CHO SI-15 cells stably transformed by the pSVIFA2-I DNA of Fig. 5 (next 4 lanes), c) CHO MEIF-5 cells expressing IFN-$\beta_1$ gene (Y. Chernajovsky et al. (1984), supra) (next 2 lanes); d) CHO cells with DHFR gene only (next lane); e) fresh medium (Non Ind.). The IFN antiviral activity measured per ml of the media added to FS11 cells is indicated. An electrophoresis of phosphatased, 32P-$\alpha$-ATP labelled (2'-5') oligo A product is shown.

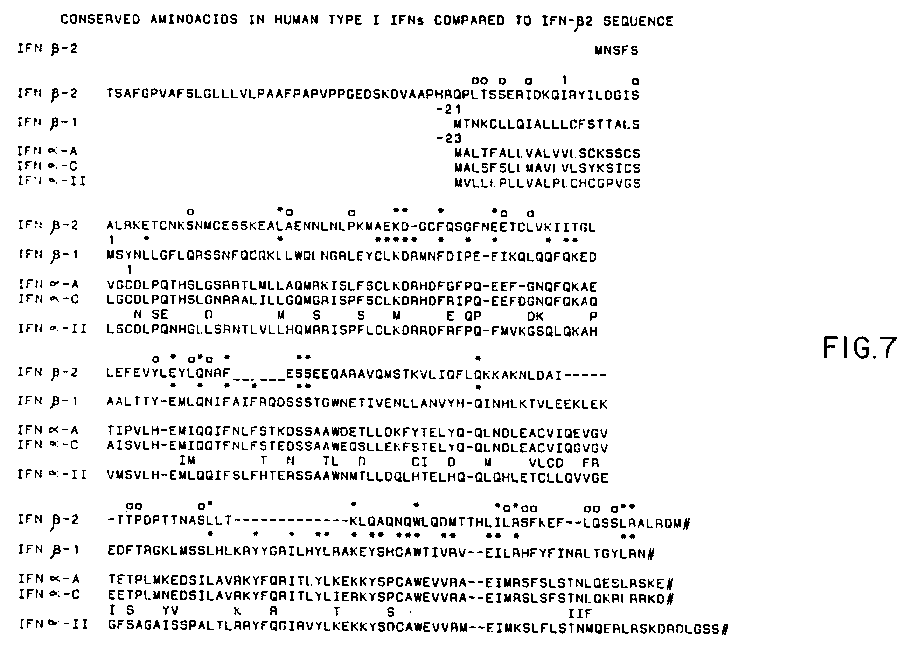

Figure 7 is a comparison of amino acid sequences between type I human IFNs. Amino acids that are conserved in IFN-$\beta_1$ and the various IFN-$\alpha$s (shown are $\alpha$A, $\alpha$C, replacements in other $\alpha$s excluding $\alpha$E, and IFN-$\alpha$ class II), are marked by stars over the IFN-$\beta_1$ sequence. Amino acids conserved in IFN-$\beta_2$ are shown above its sequence by stars if conserved in all type I IFNs, or by squares if conserved in some IFNs. IFN-$\beta_{2A}$ was aligned by comparing hydropathy plots (see text) and numbered from the presumed processing site.

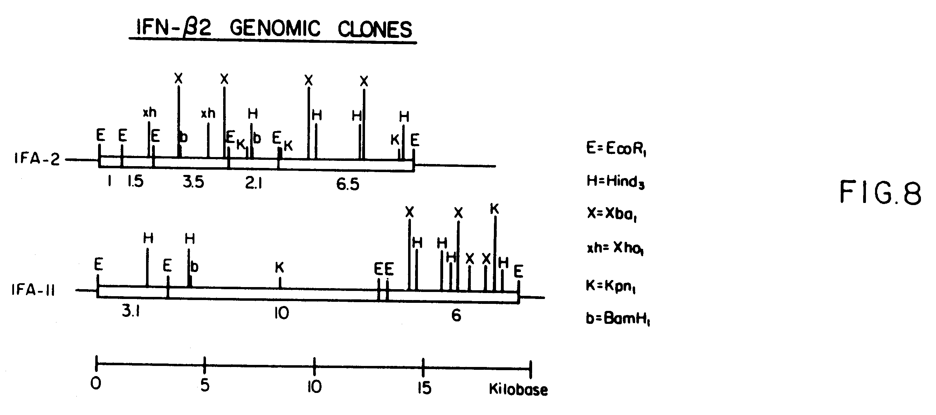

Figure 8 shows restriction maps of the two different human IFN-$\beta_2$ genes which are expressed in rodent cells. The IFA-11 DNA (IFN-$\beta_{2B}$ gene) was transfected into either CHO or L cells and expressed IFN-$\beta$ activity after induction (Table 1).

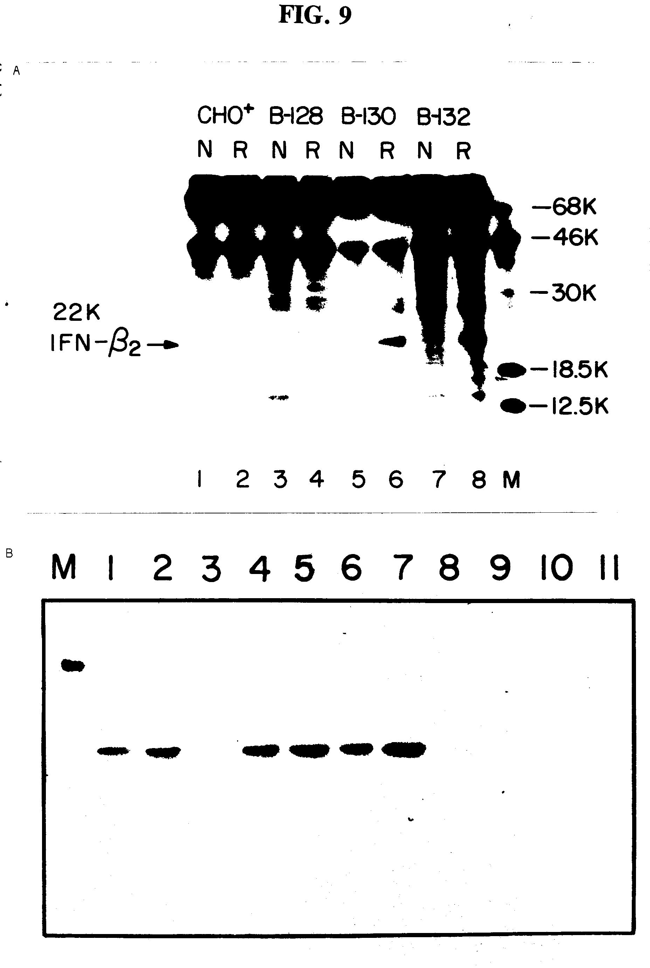

Figure 9 shows an IFN-$\beta_2$ specific immunocompetition assay with R-antibodies to show that human fibroblasts FS11 cells secrete the IFN-$\beta_2$ protein in response to the two cytokines interleukin-I (Il-I) and Tumor Necrosis Factor (TNF-$\alpha$). Both cytokines induce the synthesis and secretion of the IFN-$\beta_2$ protein in two levels which have been estimated by the immunoassay to be 10 to 20 U/ml IFN-$\beta_2$. Optimal IFN-$\beta_2$ induction was seen with the concentrations of rIL-1$\alpha$ of 4 U/ml (0.13 ng/ml). The optimal concentration of TNF-$\alpha$ for IFN-$\beta_2$ induction (400 U/ml; 40 ng/ml) was higher than that required for cytolytic effect on sensitive cells (0.1 ng/ml) and even higher than that giving optimal growth stimulation of diploid fibroblasts (2 ng/ml).

Detailed description of preferred embodiments

The existence of interferon-beta$_2$ was discovered in the course of attempts to isolate genetic material containing the nucleotide sequence coding for interferon in human fibroblast cells. In this process, fibroblast cells were induced for the production of inducible interferon mRNA with a suitable exogenous factor and then the mRNAs were extracted. At the same time the mRNAs from a non-induced culture of the same host cells were extracted. cDNA probes were synthesized from the mRNAs of both the induced culture and the non-induced control culture using the corresponding mRNAs as templates (induced cDNAs and non-induced cDNAs). Double-stranded cDNAs derived from the mRNA extracted from the induced culture were then synthesized and such cDNAs were inserted in appropriate vectors and transfected into suitable microorganisms. The microorganisms were cultivated under conditions suitable to cause selective development of microorganism colonies of the modified vectors (initial colonies). Duplicate colonies of the initial colonies were formed and the DNAs of both the initial and duplicate colonies were freed in situ. The DNAs of one of the two sets of duplicate colonies were hybridized with the cDNA probes synthesized from the mRNAs extracted from the induced culture and the other of the two sets of duplicate colonies were hybridized with the cDNA probes synthesized from the mRNAs extracted from the non-induced culture. Clones which selectively hybridized with the induced cDNA probes but not with the non-induced cDNA probes were selected and their DNAs recovered. These cloned DNAs were further tested to determine those DNAs capable of hybridizing with mRNA translatable in frog oocytes or reticulocyte-lysates into interferon, which DNAs are essentially those which code for interferon, or contain enough of the sequence coding for interferon to be used to obtain a complete interferon DNA.

During this investigation, it was discovered that two different mRNAs coding for interferon could be isolated from human fibroblast cells after being appropriately induced. Upon glycerol gradient centrifugation, the smallest mRNA sediments at 11S and yields by translation in a cell-free system a protein of molecular weight 20,000 which is selectively precipitated by antibodies prepared against one of the interferons that can be purified from these cells. This protein is human interferon-beta$_1$ of which the amino acid sequence was partially determined by E. Knight, et al. (1980) Science, 207, 525-526. The largest mRNA was found to sediment at 14S and yield a protein of molecular weight 23,000 which is precipitated by antibodies against a less purified preparation of fibroblast interferon. This protein is designated human interferon-beta$_2$. cDNA clones hybridizable to mRNA which translates to interferon-beta$_2$ were prepared in such a manner and an IFN-$\beta_2$ cDNA probe was prepared therefrom.

Two such IFN-$\beta_2$ clones A341 and E474 were found to represent overlapping sequencing and were fused to reconstitute the IFN-$\beta_{2A}$ AE20 cDNA (Figure 1). The nucleotide sequence of this IFN-$\beta_{2A}$ cDNA was determined and revealed an open reading frame of 212 amino acids predicting a protein of 23,500 daltons. Transcription-translation experiments confirmed that this cDNA encodes such a protein.

From a human gene library of human adult blood cell DNA cloned after partial EcoRI digestion in $\lambda$ charon 4A (Y. Mory et al. (1981) Eur. J. Biochem. 120: 197-202), two genomic clones IFA-2 and IFA-11 hybridizing to such an IFN-$\beta_2$ cDNA probe were isolated. Clone IFA-2 contains a gene with at least 4 exons hybridizing to various segments of the cloned cDNA (Figure 1). An Xho1 site present near the 5' of the AE20 cDNA allowed to map the genomic segment A132 (Figure 2) which contains the first IFA-2 exon ending 70 bp downstream from this Xho1 site. By S1 nuclease analysis using DNA probes labelled at a BstN1 site 40 bp after the Xho1 site, it was determined that induced FS11 human fibroblasts contain IFN-$\beta_{2A}$RNA with two distinct 5' ends S-1 and S-2 separated by 20 nucleotides (Figure 3). Each of these two starts is preceded in the IFA-2 gene by a potential TATA box at -30 (Figure 4). RNA ending at S-2 seems to be more abundant than the longer RNA under a variety of induction conditions (Figure 3). Both RNA 5' ends were also seen with cytoplasmic RNA instead of total cell RNA. Therefore, it is apparent that both RNAs are formed and active in human cells. Both IFN-$\beta_{2A}$RNAs are induced in human fibroblasts by poly (rI)(rC) and induction is stimulated by IFN priming as in the case of IFN-$\beta_1$ RNA in these cells (U. Nir et al. (1984) Nucl. Acids Res. 12: 6979-6993). A prolonged (6.5 h) treatement with CHX also induces IFN-$\beta_{2A}$RNA, but shorter (3.5 h) CHX treatment is effective only with poly (rI)(rC) (Figure 3). The low induction of IFN-$\beta_{2A}$RNA by CHX without ds RNA distinguishes this gene from IFN-$\beta_1$.

DNA constructs were made to express the IFA-2 gene under the control of the SV40 early gene promoter. A 4.8 kb segment of IFA-2 DNA was fused through a synthetic oligonucleotide to pSVE3 DNA (Y. Chernajovsky et al. (1984). supra) so that the ATG codon is 150 bp downstream from the SV40 early early RNA start (pSVIFA2-II, see legend, Figure 5). After transfection of pSVIFA2-II DNA into Cos7 monkey cells (Y. Gluzman (1981) Cell 23: 175-182), two RNAs of 1.35 and 2.2 kb were detected in Northern blots by IFN-$\beta_2$ cDNA; these two RNAs would correspond to transcripts ending at the IFN-$\beta_2$ ad SV40 polyadenylation sites respectively (Figure 5). Medium from the Cos7 cells during transient expression of the pSVIFA2-II DNA was assayed for IFN activity by inhibition of the cytopathic effect of VSV on human FS11 and Wish cells as described in D. Novick et al. (1983) J.Cen.Virol. 64: 905-910. Antiviral activity could be clearly detected two days after transfection (Table 1). The specific antiviral activity of IFN-$\beta_{2A}$per unit protein has been estimated to be 30 to 100 times lower than that of IFN-$\beta_1$ and closer to that of IFN-$\alpha_1$ (5-10 x $10^6$ units/mg). The IFN titers observed by transient expression of the IFA-2 gene were as expected, about 3% of those seen with the IFN-$\beta_1$ gene under the same SV40 promoter control (Table 1). Production of IFN-$\beta_{2A}$activity by pSVIFA2-II DNA cells was further demonstrated by induction of (2'-5') oligo A synthetase (M. Revel et al. (1981), supra) in FS11 cells exposed to the medium of the transfected Cos7 cells (Figure 6). The induction was dose-dependent and corresponded quantitatively to the antiviral activity.

The IFA-2 gene was also fused to the SV40 early promoter directly at the Xho1 site near S-2 (pSVIFA2-I DNA, legend of Figure 5). Hamster CHO-K1 DHFR⁻ cells were cotransformed with pSVIFA2-I and pSVDHFR (Y. Chernajovsky et al. (1984), supra) DNAs and a stably transformed line SI-15 was isolated. This cell line produces constitutively 10-50 U/ml of IFN antiviral activity measured on human FS11 and Wish cells, while CHO cells transformed only by pSVDHFR produce no human IFN (Table 1). By concentration of the medium from SI-15 cultures, rIFN-$\beta_{2A}$solutions titrating about 3000 U/ml could regularly be obtained. This rIFN-$\beta_{2A}$has the properties expected for a human IFN and induces the (2'-5') oligo A synthetase in FS11 cells even at low concentrations of 1 U/ml (Figure 6). The rIFN-$\beta_{2A}$constitutively produced by CHO SI-15 cells demonstrated human-specific induction of the mRNAs for (2'-5') oligo A synthetase, C56 and HLA in the presence of cycloheximide, its antiviral activity was neutralized by anti-IFN-$\beta_1$ (but not anti-IFN-$\alpha$ or anti-IFN -$\gamma$ ) polyclonal and monoclonal antibodies and it acted on mouse-human hybrids only if they contain human chromosome 21 which carries the gene for the type 1 IFN receptor. These further characterizations of the biological activity of rIFN-$\beta_{2A}$ convincingly demonstrate that it acts directly as an IFN and is not an inducer of IFN-$\beta_1$.

Improved yields of rIFN-$\beta_{2A}$were obtained with DNA constructs containing the IFN-$\beta_{2A}$ cDNA sequence fused to the SV40 early promotor (pSVCIF$\beta$2 shown in Figure 5), when this DNA cotransfected with pSVDHFR into CHO cells as above, was amplified by selecting cells resistant to increasing concentrations of methotrexate. Without amplification, the pSVCIF$\beta_{2A}$transformed CHO cells (e.g. clone B-132) produced levels of IFN-$\beta_{2A}$activity similar to those produced by the SI-15 cells (Table 2). However, after selection for resistance to methotrexate, higher yields were obtained with up to 800 U/ml (Table 2).

From such clones it was possible to immunoprecipitate the IFN-$\beta_{2A}$ protein secreted into the culture media and if the cells had been labelled by $^{35}$S-methionine to analyse the IFN-$\beta_{2A}$protein by dodecyl sulphate polyacrylamide gel electrophoresis. The size of the secreted rIFN-$\beta_{2A}$was found to be 21,000 daltons. Indeed, experiments have shown that the 23-26 Kd primary translation product of IFN-$\beta_{2A}$RNA in reticulocyte lysates is processed in vitro by dog pancreatic membranes into a shortened 21 Kd protein, and that induced human fibroblasts produce a 21 Kd protein which is immunologically similar to the IFN-$\beta_{2A}$RNA 26 Kd product, but distinct from IFN-B$_1$. The N-terminus of the mature IFN-$\beta_{2A}$21 Kd protein has not been determined and two potential glycosylation sites are present in the IFN-$\beta_{2A}$sequence (Figure 1) making it difficult to calculate the size of the region removed by processing. However, the decrease in size indicates that this processed region is longer than that in IFN-$\beta_1$, the size of which increases by maturation (W. Degrave et al. (1981) Gene 10: 11-15). A hydropathy plot of the IFN-$\beta_{2A}$26 Kd polypeptide has shown that there is a marked similarity between hydrophobic and hydrophilic regions of IFN-$\beta_{2A}$and of mature IFN-$\beta_1$, when the two proteins are aligned by their C-termini. The same alignment also reveals conservation in the amino acid sequence of IFN-$\beta_{2A}$with those of the other type I human IFNs (Figure 7). Between all the known IFN- $\alpha$s and $\beta_1$ sequences, there are 38 amino acids which are conserved (marked by stars in Figure 7). When IFN-$\beta_{2A}$is aligned is above, 18 of these 38 amino acids common to all type I human IFNs can be seen to be conserved (Figure 7). There is an overall homology of about 20% between IFN-$\beta_{2A}$and the other type I IFNs.

Although the sequence homology between IFN-$\beta_1$ and $\beta_{2A}$ is low, the cross-neutralization of the antiviral activities of IFN-$\beta_1$ and IFN-$\beta_{2A}$ by the same antibodies, including a monoclonal anti-IFN-$\beta_1$ (D. Novick et al. (1983), supra), could result from some conserved structure in the active site of IFN-$\beta$. IFN-$\beta_{2A}$activity is not neutralized by anti-IFN-$\alpha$ or $\gamma$ antibodies, justifying its designation as a $\beta$-type IFN. The genes encoding IFN-$\beta_2$ are not on chromosome 9 (U. Nir (1984), supra) and differ from the type I IFN genes in the chromosome 9 cluster by the presence of introns. The IFA-2 gene was determined to be on chromosome 7 (P-B. Sehgal et al. (1986) Proc.Natl.Acad.Sci. USA, in press). The amino acid sequence homology detected is compatible with the notion that the IFN-$\beta_2$ genes are related to the ancestral gene which gave rise to the type I IFN gene cluster.

The second genomic DNA clone IFA-11 hybridizing with the IFN-$\beta_{2A}$cDNA has a restriction map which differs from that of IFA-2 (Figure 8). Transcript mapping and sequencing showed that the 3' exon of IFA-2 and IFA-11 (Xbal-HindIII segment) are identical (99% sequence homology), while there was no cross-hybridization under stringent conditions between more 5' exons. The IFA-11 genomic clone (19 Kb) was transfected into L-TK⁻ cells together with a HSV-TK gene (F. Colbere-Garapin et al. (1981) J.Mol.Biol. 150: 1-14) and stable transformants were isolated. Table 1 shows that L cell clone LI-39 containing the human IFA-11 gene, produces human IFN activity when induced by poly (rI)(rC) and cycloheximide, while L-TK$^+$ cells produced no such activity. A CHO clone 1C40 containing the IFA-11 gene also produced human IFN activity (Table 1). No activity was seen in non-induced LI-39 or 1C40 cells. The IFN activity obtained with IFA- 11 transformants after induction was significantly higher than with the IFA-2 gene. The rIFN-$\beta_{2B}$ produced by expression of the IFA-11 gene was neutralized with anti-IFN-$\beta_1$ similarly to the IFA-2 gene product. IFA-11 specific DNA probes hybridized to RNA from induced human fibroblasts similarly to IFN-2 DNA probes, indicating that both genes are active in human cells.

The function of the IFN-$\beta_2$ genes seems to result from their expression under conditions in which other IFN genes are not expressed. We find that fibroblasts exposed to Tumor Necrosis Factor (TNF) or to lymphokine IL-1 produce the IFN-$\beta_2$ protein. Others found that neutralization of IFN-$\beta$ by antibodies results in a stimulation of cell proliferation (M. Kohase et al. (1986) Cell, in press; D. Resnitzky et al. (1986) Cell, in press), suggesting that the IFN-$\beta_2$ acts to limit cell growth in response to growth factors. Supporting this conclusion is the fact that RNA hybridizing to IFN-$\beta_2$ cDNA was recently detected in peripheral blood mononuclear cells induced by mitogens such as PHA along with IFN-$\gamma$ mRNA. It has also been reported that an IL-1 like lymphokine strongly induces RNA hybridizing to IFN-$\beta_2$ cDNA in diploid fibroblasts and other human cell lines (J. Content et al. (1985) Eur.J.Biochem. 152: 253-257). The promoter region of the IFA-2 genes seems to contain two TATA boxes and two RNA starts (Figures 1 and 3). This IFN-$\beta_2$ promoter was shown to respond to either poly (rI)(rC) or to cycloheximide (Y. Chernajovsky et al. (1984), supra). In vivo, the two IFN-$\beta_2$ gene promoters may be activated by different inducers (ds RNA, protein synthesis inhibitors, IL-1, PHA). A variety of cells have been found to produce autocrine IFN-$\beta$ in low amounts during growth arrest and differentiation (M. Revel (1983) Interferon 5 pp.205-239, Acad. Press, London). IFN-$\beta_2$ may belong to the same group of minor IFN species produced by cells to auto-regulate their growth, while the classical type I IFNs are probably more specifically involved in theresponse to viral infections.

The biological significance of IFN-$\beta_2$ lies most probably in the fact that it is induced under conditions where IFN-$\beta_1$ is not induced, as in metabolically stressed cells (A. Zilberstein et al., (1985) in The Interferon System, Serono Symposia Raven Press, pp.73-83). Most important is the observation that TNF induces IFN-$\beta_2$ in fibroblasts and that the proliferation of these cells which is stimulated by TNF is further enhanced if anti-IFN-$\beta$ antibodies are added. A number of human cell lines have been shown to produce autocrine IFN-$\beta$ species when undergoing differentiation, and these IFNs are responsible for the induction of HLA antigens on these cells (A. Yarden et al. (1984) Embo.J. 3: 969-973). In a mouse myeloleukemic cell line induced to differentiate by CSF-1, the growth arrest of the cells which characterizes terminal differentiation, is abolished by anti-IFN-$\beta$ antibodies. IL-1 stimulates growth of several cell types and the classical growth factor PDGF was found to induce IFN-$\beta$ RNA in mouse cells (J.N. Zullo et al. (1985) Cell 43: 793-800). Although multiple IFN-$\beta$ species may be involved (P.B. Sehgal, (1982) in Interferon 4, Academic Press, pp.1-22, and M. Revel (1983) in Interferon 5, Academic Press, pp. 205 - 239), IFN-$\beta_2$ appears to be one of these autocrine autoregulators of cell growth produced in response to growth factors. This would be in line with the low specific activity of IFN-$\beta_2$ (estimated by comparison of immunoassay and antiviral activity to be 50-100 times lower than for IFN-$\beta_1$), since only very small antiviral activity is seen in cells producing the autocrine IFN-$\beta$ species (M. Revel (1983) in Interferon 5, Academic Press, pp. 205-239). This could also explain why the hamster cells expressing IFN-$\beta_2$ cDNA produce less IFN activity than similar CHO cells harbouring the human IFN-$\beta_1$ gene (Y. Chernajovsky et al. (1984), DNA 3: 297-308). Interestingly, the IFN-$\alpha$ 1 (D) species has also a 100 times lower specific activity on human cells than other IFN-$\alpha$ species (T. Goren et al. (1983), Virology 130: 273-280), although it represents a large proportion of the total mass of leukocyte IFN (C. Weissmann (1981) in Interferon 3, Academic Press, N.Y. pp.101-134). It is not excluded that some of the multiple IFN functions will be expressed more efficiently by IFN-$\beta_2$ than the antiviral effect. Despite their low concentration, growth-regulatory IFN-$\beta$ induce HLA and (2'-5') oligo A synthetase more strongly than expected, have more prolonged effects and markedly inhibit the growth of the cells which produce them. The induction of IFN-$\beta_2$ by IL-1 and TNF suggests that it may play a role as an autocrine mediator of some effects of these cytokines in inflammation and acute-phase responses, as well as regulating cell proliferation.

Induction of mRNA which hybridizes with IFN-$\beta_2$ cDNA probes but not to IFN-$\beta_1$ cDNA has been observed in human cell lines after exposure to Interleukin-1 (IL-1) (J. Content et al. (1985), Eur.J.Biochem. 152: 253-257) and Tumor Necrosis Factor (TNF- $\alpha$ ). The IFN-$\beta_2$-specific immunocompetition assay with R-antibodies was used to examine if human fibroblasts FS11 cells indeed secrete the IFN-$\beta_2$ protein in response to these two cytokines (Figure 9). Both rIL-1$\alpha$ and TNF-$\alpha$ induced the synthesis and secretion of the IFN-$\beta_2$ protein, to levels estimated by the immunoassay to be 10-20 U/ml IFN-$\beta_2$. Optimal IFN-$\beta_2$ induction was seen with concentrations of rIL-1$\alpha$ of 4 U/ml (0.13 ng/ml), similar to those required for IL-1 induction of prostaglandin E$_2$, collagenase and hyaluronic acid in human fibroblasts (J.H. Korn (1985), Arthritis Rheum. 28: 315-322). The optimal concentration of TNF-$\alpha$ for IFN-$\beta_2$ induction (400 U/ml; 40 ng/ml) was higher than that required for the cytolytic effect on sensitive cells (0.1 ng/ml; A.M. Wang et al. (1985) Science 228: 149-154) and even higher than that giving optimal growth stimulation of diploid fibroblasts (2 ng/ml). The onset of IFN-$\beta_2$ secretion was somewhat faster with TNF-$\alpha$ reaching half maximal levels at about 6 hours, versus 8-12 hours with IL-1.

To the extent that it may be demed necessary in order to complete the disclosure in this case; all of the subject matter of each of the publications referred to and cited hereinabove are hereby incorporated herein by reference.

It will be obvious to those skilled in the art that various other changes and modifications may be made without departing from the scope of the invention and the invention is not to be considered limited to what is shown in the drawing and described in the specification.

TABLE 1

| Antiviral Activity of rIFN-$\beta_2$* Produced in Transfected Mammalian Cells I. IFA-2 GENE<br>A. Constitutive expression in CHO cells | |
|---|---|
| pSVIFA2-I S1-15 cells | 10-50 U/ml** |
| (control CHO cells | 0 ) |
| B. Transient expression in Cos7 cells | |
| pSVIFA2-II DNA | 15 U/ml |
| (control IFN-$\beta_1$ pSVEIF DNA | 500 U/ml) |
| II. IFA-11 GENE<br>A. Induced expression*** in CHO cells | |
| Clone 1C40 | 400-500 U/ml |
| (control CHO cells | 100 U/ml) |
| B. Induced expression in L cells | |
| Clone LI-39 | 250 U/ml |
| (control L-TK$^+$ cells | <4 U/ml) |

\* Assay with VSV in FS11 and WISH cells. Neutralized by anti-IFN-$\beta_1$.
\*\* 3000 U/ml after concentration.
\*\*\* Induction with poly IC and cycloheximide for 10 hrs. Harvest at 28 hrs.

TABLE 2

| Antiviral Activity of Human rIFN-$\beta_{2A}$ Produced in Hamster Cells Transfected and Amplified with IFN-$\beta_{2A}$ cDNA | | |
|---|---|---|
| CHO-SVCIF$\beta$2 B132-10M | CLONE: | Human IFN activity* U/ml |
| B-132 | (0 MTX)** | 64 |
| B-132-5M | (250 nM MTX)** | 300 |
| B-132-10M | (500 nM MTX)** | 800 |

\* Medium from cultures was collected every 24 hours and assayed with VSV in FS11 human cells. The activity was neutralized by anti-IFN-$\beta_1$, but not anti-IFN-$\alpha$ or anti-IFN-$\gamma$.
\*\* Cultures were selected for resistance to indicated concentrations of methotrexate.

**Claims**

1. Use of human interferon-$\beta_{2A}$ for preparing a medicament for treatment of diseases in the fields of inflammation and/or acute phase response.

2. Use according to claim 1, wherein IFN-$\beta_{2A}$ inhibits fibroblast proliferation.

3. Use according to claim 1, wherein IFN-$\beta_{2A}$ prevents sclerosis after infection.

**Patentansprüche**

1. Verwendung von menschlichem Interferon-$\beta_{2A}$ zur Herstellung eines Medikaments zur Behandlung von Krankheiten auf den Gebieten der Entzündung und/oder Akute-Phase-Antwort.

2. Verwendung nach Anspruch 1, worin IFN-$\beta_{2A}$ die Fibroblasten-Proliferation hemmt.

3. Verwendung nach Anspruch 1, worin IFN-$\beta_{2A}$ Sklerose nach der Infektion verhindert.

**Revendications**

1. Utilisation d'interféron-$\beta_{2A}$ humain pour préparer un médicament pour le traitement de maladies dans les domaines d'inflammation et/ou de réponse phase aigue.

2. Utilisation selon la revendication 1, dans laquelle IFN-$\beta_{2A}$ inhibe la prolifération des fibroblastes.

3. Utilisation selon la revendication 1, dans laquelle IFN-$\beta_{2A}$ prévient une sclérose après une infection.

# FIG. 1 (PART 1)

IFN-β2a cDNA

IFN-Beta 2a cDNA

```
              10          20          30          40          50          60
              •           •           • - Xhol -   •           •           •
AG GAC TGG AGA TGT CTG AGG CTC ATT CTG CCC TCG AGC CCA CCG GGA ACG AAA GAG AAG CTC
              70          80          90         100         110         120
              •           •         - •  Xmn1 -   •           •           •
TAT CTC CCC TCC AGG AGC CCA GCT ATG AAC TCC TTC TCC ACA AGC GCC TTC GGT CCA GTT
                                    Met Asn Ser Phe Ser Thr Ser Ala Phe Gly Pro Val
                                     1                                          10
             130         140         150         160         170         180
              •           •           •           •           •           •
GCC TTC TCC CTG GGG CTG CTC CTG GTG TTG CCT GCT GCC TTC CCT GCC CCA GTA CCC CCA
Ala Phe Ser Leu Gly Leu Leu Leu Val Leu Pro Ala Ala Phe Pro Ala Pro Val Pro Pro
                          20                                          30
             190         200         210         220         230         240
              •           •           •           •           •           •
GGA GAA GAT TCC AAA GAT GTA GCC GCC CCA CAC AGA CAG CCA CTC ACC TCT TCA GAA CGA
Gly Glu Asp Ser Lys Asp Val Ala Ala Pro His Arg Gln Pro Leu Thr Ser Ser Glu Arg
                          40                                          50
             250         260         270         280         290         300
              •           •           •           •           •           •
ATT GAC AAA CAA ATT CGG TAC ATC CTC GAC GGC ATC TCA GCC CTG AGA AAG GAG ACA TGT
Ile Asp Lys Gln Ile Arg Tyr Ile Leu Asp Gly Ile Ser Ala Leu Arg Lys Glu Thr Cys
                          60                                          70
             310         320         330         340         350         360
              •           •           •           •           •           •
AAC AAG AGT AAC ATG TGT GAA AGC AGC AAA GAG GCA CTG GCA GAA AAC AAC CTG AAC CTT
Asn Lys Ser Asn Met Cys Glu Ser Ser Lys Glu Ala Leu Ala Glu Asn Asn Leu Asn Leu
                          80                                          90
             370         380         390         400         410         420
              •           •           •           •           •           •
CCA AAG ATG GCT GAA AAA GAT GGA TGC TTC CAA TCT GGA TTC AAT GAG GAG ACT TGC CTG
Pro Lys Met Ala Glu Lys Asp Gly Cys Phe Gln Ser Gly Phe Asn Glu Glu Thr Cys Leu
                         100                                         110
             430         440         450         460         470         480
              •           •           •           •           •           •
GTG AAA ATC ATC ACT GGT CTT TTG GAG TTT GAG GTA TAC CTA GAG TAC CTC CAG AAC AGA
Val Lys Ile Ile Thr Gly Leu Leu Glu Phe Glu Val Tyr Leu Glu Tyr Leu Gln Asn Arg
                         120                                         130
             490         500         510         520         530         540
              •           •           •           •           •           •
TTT GAG AGT AGT GAG GAA CAA GCC AGA GCT GTC CAG ATG AGT ACA AAA GTC CTG ATC CAG
Phe Glu Ser Ser Glu Glu Gln Ala Arg Ala Val Gln Met Ser Thr Lys Val Leu Ile Gln
                         140                                         150
             550         560         570         580         590         600
              •           •         -Xba1• -      •           •           •
TTC CTG CAG AAA AAG GCA AAG AAT CTA GAT GCA ATA ACC ACC CCT GAC CCA ACC ACA AAT
Phe Leu Gln Lys Lys Ala Lys Asn Leu Asp Ala Ile Thr Thr Pro Asp Pro Thr Thr Asn
                         160                                         170
             610         620         630         640         650         660
              •           •           •           •           •           •
GCC AGC CTG CTG ACG AAG CTG CAG GCA CAG AAC CAG TGG CTG CAG GAC ATG ACA ACT CAT
Ala Ser Leu Leu Thr Lys Leu Gln Ala Gln Asn Gln Trp Leu Gln Asp Met Thr Thr His
                         180                                         190
             670         680         690         700         710         720
              •           •           •           •           •           •
CTC ATT CTG CGC AGC TTT AAG GAG TTC CTG CAG TCC AGC CTG AGG GCT CTT CGG CAA ATG
Leu Ile Leu Arg Ser Phe Lys Glu Phe Leu Gln Ser Ser Leu Arg Ala Leu Arg Gln Met
                         200                                         210
             730         740         750         760         770         780
              •           •           •           •           •           •
TAG CAT GGG CAC CTC AGA TTG TTG TTG TTA ATG GGC ATT CCT TCT TCT GGT CAG AAA CCT
             790         800         810         820         830         840
              •           •           •           •           •           •
GTC CAC TGG GCA CAG AAC TTA TGT TGT TCT CTA TGG AGA ACT AAA AGT ATG AGC GTT AGG
             850         860         870         880         890         900
              •           •           •        -Aha3 •-       •           •
ACA CTA TTT TAA TTA TTT TTA ATT TAT TAA TAT TTA AAT ATG TGA AGC TGA GTT AAT TTA
             910         920         930         940         950         960
              •           •           •           •           •           •
TGT AAG TCA TAT TTT ATA TTT TTA AGA AGT ACC ACT TGA AAC ATT TTA TGT ATT AGT TTT
             970         980         990        1000        1010        1020
              •           •           •           •           •           •
GAA ATA ATA ATG GAA AGT GGC TAT GCA GTT TGA ATA TCC TTT GTT TCA GAG CCA GAT CAT
            1030        1040        1050        1060        1070        1080
              •           •           •           •           •           •
                                                                     -Aha3   •
TTC TTG GAA AGT GTA GGC TTA CCT CAA ATA AAT GGC TAA CTT TAT ACA TAT TTT TAA AGA
            1090        1100        1110        1120        1130        1140
  -           •           •           •           •           •           •
AAT ATT TAT ATT GTA TTT ATA TAA TGT ATA AAT GGT TTT TAT ACC AAT AAA TGG CAT TTTAAA...
```

# FIG. 1 (PART 2)

STRUCTURE OF HUMAN IFNβ2 GENE IFA-2

FIG.2

# FIG.3

IFN-β2 Gene, Clone IFA-2

<pre>
        -150       -140        -130        -120        -110       -100
IFA-2   ACTAAAGGACGTCACATTGCACAATCTTAATAAGGTTTCCAATCAGCCCCACCCGCTCTG

        -90        -80         -70         -60         -50        -40
IFA-2   GCCCCACCCTCACCCTCCAACAAAGATTTATCAAATGTGGGATTTTCCCATGAGTCTCAA

        -30        -20         -10          1          10         20
IFA-2   TATTAGAGTCTCAACCCCCAATAAATATAGGACTGGAGATGTCTGAGGCTCATTCTGCCC
cDNA    *****                    ******      -------..----------ATTCTGCCC
        TATA-1                   TATA-2      S-1                 S-2

        30xho1     40          50          60          70         80
IFA-2   TCGAGCCCACCGGGA-CGAAAGAGAAGCTCGATCTCCCCTCCAGGAGCCCAGCTATGAAC
cDNA    TCGAGCCCACCGGGAACGAAAGAGAAGCTCTATCTCCCCTCCAGGAGCCCAGCTATGAAC
                                                                    X
        90         100    intr1 110        120         130        140
IFA-2   TCCTTCTCCACAA GTAAGTGCAGGAAATCCTTAGCCCTGAACTGCCAGCGGGGTCGAGCC
cDNA    TCCTTCTCCACAA GCGCCTTCGCTCCAGTTGCCTTCTCCCTGGGGCTGCTC....

        150        160         170         180         190        200
IFA-2   CTGTGTGAGGGAGGGGTGTGTGGCCCAAGGAGGGCTGGCGGGCGGCCAGCAGCAGAGGCA

        210        220         230         240         250
IFA-2   GGCTCCCAGCTGTGCTGTCAGCTCACCCTGCGCTCGCTCCCTCGC....
</pre>

FIG. 4

EP 0 536 520 B1

CONSTITUVE EXPRESSION OF Hu IFN β2 IN CHO CELLS

FIG.5

clone B131 250nMMTX = 300 UNITS/ML   (VSV, Mengo)

EP 0 536 520 B1

INDUCTION OF 2'-5' OLIGO A SYNTHETASE BY FIG.6
r-IFN-β2 FROM GENE TRANSFECTED CELLS

PRODUCT OF          PRODUCT OF        IFN-β1   CHO   NON
pSVIFA2-II          pSVIFA2-I          CHO    cell   IND.
in COS7 cells       in CHO cells      cells  controls
                    antiviral units
1   5   10  20    1   5   10  20      1   20    0     0

2'-5'ApApA —

2'-5' ApA —

EP 0 536 520 B1

CONSERVED AMINOACIDS IN HUMAN TYPE I IFNs COMPARED TO IFN-β2 SEQUENCE

```
IFN β-2                                                                              MNSFS


                                              oo  o   o    1           o
IFN β-2      TSAFGPVAFSLGLLLVLPAAFPAPVPPGEDSKDVAAPHRQPLTSSERIDKQIRYILDGIS
                                               -21
IFN β-1                                        MTNKCLLQIALLLCFSTTALS
                                               -23
IFN ∝-A                                        MALTFALLVALVVLSCKSSCS
IFN ∝-C                                        MALSFSLIMAVIVLSYKSICS
IFN ∝-II                                       MVLLLPLLVALPLCHCGPVGS



                      o          *o        o      **     *      *o  o
IFN β-2      ALRKETCNKSNMCESSKEALAENNLNLPKMAEKD-GCFQSGFNEETCLVKIITGL
             1     *                    *      *****  *   *   *       *  **
IFN β-1      MSYNLLGFLQRSSNFQCQKLLWQINGRLEYCLKDRMNFDIPE-EIKQLQQFQKED
              1
IFN ∝-A      VGCDLPQTHSLGSRRTLMLLAQMRKISLFSCLKDRHDFGFPQ-EEF-GNQFQKAE
IFN ∝-C      LGCDLPQTHSLGNRRALILLGQMGRISPFSCLKDRHDFRIPQ-EEFDGNQFQKAQ
                  N SE   D       M   S   S   M     E QP    DK     P
IFN ∝-II     LSCDLPQNHGLLSRNTLVLLHQMRRISPFLCLKDRRDFRFPQ-FMVKGSQLQKAH



                o * o*o *               **                    *
IFN β-2      LEFEVYLEYLQNRF___ _____ESSEEQARAVQMSTKVLIQFLQKKAKNLDAI-----
                  *  *  * *    **       *                     *
IFN β-1      AALTTY-EMLQNIFAIFRQDSSSTGWNETIVENLLANVYH-QINHLKTVLEEKLEK

IFN ∝-A      TIPVLH-EMIQQIFNLFSTKDSSAAWDETLLDKFYTELYQ-QLNDLEACVIQEVGV
IFN ∝-C      AISVLH-EMIQQTFNLFSTEDSSAAWEQSLLEKFSTELYQ-QLNDLEACVIQGVGV
                         IM    T  N  TL  D      CI  D    M    VLCD  FR
IFN ∝-II     VMSVLH-EMLQQIFSLFHTERSSAAWNMTLLDQLHTELHQ-QLQHLETCLLQVVGE



                oo         o*                   *        *        *o*oo      oo o**
IFN β-2      -TTPQPTTNASLLT------------KLQAQNQWLQDMTTHLILRSFKEF--LQSSLRALRQM#
                           *    *    *   **   *  **  ***   **    **  *    **
IFN β-1      EDFTRGKLMSSLHLKRYYGRILHYLRAKEYSHCAWTIVRV--EILRHFYFINRLTGYLRN#

IFN ∝-A      TETPLMKEDSILAVRKYFQRITLYLKEKKYSPCAWEVVRA--EIMRSFSLSTNLQESLRSKE#
IFN ∝-C      EETPLMNEDSILAVRKYFQRITLYLIERKYSPCAWEVVRA--EIMRSLSFSTNLQKRLRKD#
                 I S  YV    k   R     T   S                        IIF
IFN ∝-II     GFSAGAISSPALTLRRYFQGIRVYLKEKKYSDCAWEVVRM--EIMKSLFLSTNMQERLRSKDRDLGSS#
```

FIG.7

IFN-β2 GENOMIC CLONES

FIG.8

E = EcoR₁
H = Hind₃
X = Xba₁
xh = Xho₁
K = Kpn₁
b = BamH₁

# FIG. 9